# EUROPEAN PATENT SPECIFICATION

(11) **EP 1 513 464 B1**
(45) Date of publication and mention of the grant of the patent: **09.08.2006**
(21) Application number: 03739020.0
(22) Date of filing: 03.06.2003
(51) Int. Cl.: A61B 18/14

(54) **LAPAROSCOPIC BIPOLAR ELECTROSURGICAL INSTRUMENT**
BIPOLARES ELEKTROCHIRURGISCHES LAPAROSKOPIEINSTRUMENT
INSTRUMENT ELECTROCHIRURGICAL BIPOLAIRE LAPAROSCOPIQUE

(30) Priority: 06.06.2002 US 164654
(43) Date of publication of application: 16.03.2005
(60) Divisional application: 06005185.1
(73) Proprietor: Sherwood Services AG, 8200 Schaffhausen (CH)
(72) Inventor: BUYSSE, Steven, P., Longmont, CO 80501 (US); LAWES, Kate, R., Superior, CO 80027 (US); SCHMALTZ, Dale, F., Fort Collins, CO 80524 (US); LANDS, Michael, J., Louisville, CO 80027 (US); LUKIANOW, S., Wade, Boulder, CO 80305 (US); JOHNSON, Kristin, D., Louisville, CO 80027 (US); Couture, Gary M., Longmont, CO 80501 (US); NGUYEN, Lap, P., Longmont, CO 80501 (US)
(74) Representative: HOFFMANN EITLE
(86) International application number: PCT/US2003/017335
(87) International publication number: WO 2004/052221

(56) References cited:
- DE-C- 19 608 716
- US-A- 4 492 231
- US-A- 5 509 922

## Description

### BACKGROUND

### Field of the Invention

This invention relates to an electrosurgical instrument for performing laparoscopic surgical procedures, and more particularly to a laparoscopic electrosurgical instrument that is capable of grasping vessels and vascular tissue with sufficient force between two bipolar jaws to seal the vessel or vascular tissue.

### Background of Related Art

U.S. Patent No. 5,509,922 describes an endoscopic surgical instrument. The instrument comprises a handle assembly with a stationary handle and a pivoting handle, and a body portion with an outer tubular member. A lower jaw member and an upper jaw member are attached to a distal end of the body portion. By operating the handle assembly, the jaw members are pivoted about a pivot point to effect their opening and closing. The stationary handle comprises a connection port for providing the necessary current to the jaw members and therewith to respective tool members. A pair of stop members is provided to limit the rotational movement of the pivoting handle about the pivot point. A locking mechanism may be utilized to position the handles at various locations during the opening and closing procedure, which allow for the application of various closing forces on the tool member at the distal end of the instrument.

U.S. Patent No. 4,492,231 relates to a non-sticking electrocautery system and bipolar electrocautery forceps. The forceps includes a pair of electrically conductive arms terminating in respective jaws adapted to grasp tissue to be cauterized.

Laparoscopic surgical instruments are used to perform surgical operation without making large incisions in the patient. The laparoscopic instruments are inserted into the patient through a cannula, or port, that has been made with a trocar. Typical sizes for cannulas range from three millimeters to twelve millimeters. Smaller cannulas are usually preferred, and this presents a design challenge to instrument manufacturers who must find ways to make surgical instruments that fit through the cannulas.

Certain surgical procedures require cutting blood vessels or vascular tissue. This sometimes presents a problem for surgeons because it is difficult to suture blood vessels using laparoscopic tools. Very small blood vessels, in the range below two millimeters in diameter, can often be closed using standard electrosurgical techniques. If a larger vessel is severed, it may be necessary for the surgeon to convert the laparoscopic procedure into an open-surgical procedure and thereby abandon the benefits of laparoscopy.

Several journal articles have disclosed methods for sealing small blood vessels using electrosurgery. An article entitled Studies on Coagulation and the Development of an Automatic Computerized Bipolar Coagulator, J. Neurosurg., Volume 75, Jul. 1991, describes a bipolar coagulator which is used to seal small blood vessels. The article states that it was not possible to safely coagulate arteries with a diameter larger than 2 to 2.5 mm. A second article is entitled Automatically Controlled Bipolar Electrocoagulation--"COA-COMP", Neurosurg. Rev. (1984), pp. 187-190. This article describes a method for terminating electrosurgical power to the vessel so that charring of the vessel walls can be avoided.

It has been recently determined that electrosurgical methods may be able to seal larger vessels using an appropriate electrosurgical power curve, coupled with an instrument capable of applying a large closure force to the vessel walls. It is thought that the process of coagulating small vessels is fundamentally different than electrosurgical vessel sealing. Coagulation is defined as a process of desiccating tissue wherein the tissue cells are ruptured and dried. Vessel sealing is defined as the process of liquefying the collagen in the tissue so that it cross-links and reforms into a fused mass. Thus, coagulation of small vessels is sufficient to permanently close them. Larger vessels need to be sealed to assure permanent closure.

It would be desirable to have a surgical tool capable of applying electrosurgical energy, capable of applying a large closure force to the vessel walls, and also capable of fitting through a cannula. A large closure force between the jaws typically requires a large moment about the pivot for each jaw. This presents a challenge because the first and second pins have a small moment arm with respect to the pivot of each jaw. A large force, coupled with a small moment arm, is undesirable because the large forces may shear the first and second pins. It is also undesirable to increase the moment arm of the first and second pins because the physical size of the yoke might not fit through a cannula.

Several bipolar laparoscopic instruments are known. For example, U.S. Pat. No, 3,938,527 discloses a bipolar laparosoopic instrument for tubal cauterization. U.S. Pat. No. 5,250,047 discloses a bipolar laparoscopic instrument with a replaceable electrode tip assembly. U.S. Pat No. 5,445,638 discloses a bipolar coagulation and cutting forceps with first and second conductors extending from the distal end. U.S. Pat. No. 5,391,166 discloses a bipolar endoscopic instrument having a detachable working end. U.S. Pat. No, 5,342,359 discloses a bipolar coagulation device.

The present invention solves the problem of providing a large closure force between the jaws of a laparoscopic bipolar electrosurgical instrument, using a compact design that fits through a cannula, without risking structural failure of the instrument yoke.

### SUMMARY OF THE INVENTION

The present invention, which is defined in claim 1 below, relates to a laparoscopic bipolar electrosurgical instrument for sealing tissue and includes a handle having an elongated tube affixed thereto. The tube includes first and second jaw members attached to a distal end thereof which are movable from a first position for approximating tissue to at least one subsequent position for grasping tissue therebetween. Each of the jaw members includes an electrically conductive sealing surface. The handle has a fixed handle and a handle which is movable relative to the fixed handle to effect movement of the jaw members from the first position to the at least one subsequent position for grasping tissue. The jaw members are connected to a source of electrosurgical energy such that the jaw members are capable of conducting bipolar electrosurgical energy through the tissue held therebetween. A stop is included for maintaining a minimum separation distance between opposing sealing surfaces and a ratchet is included for maintaining a closure force in the range of about 3 kg/cm² to about 16 kg/cm² between opposing sealing surfaces. As can be appreciated, the stop member advantageously creates a minimum separation between electrically conductive opposing sealing surfaces to effect an efficient, consistent and uniform tissue seal.

The stop maintains a minimum separation distance of at least 0.03 millimeters between opposing sealing surfaces. Advantageously, the stop maintains a minimum separation distance of about 0.03 millimeters to about 0.16 millimeters. The stop may be disposed on at least one of the electrically conductive sealing surfaces, or alternatively, the stop may be located adjacent one of the electrically conductive sealing surfaces. Although it is preferable to locate the stop member on one or both of the electrically conductive opposing sealing surfaces, it may in some instances be advantageous to locate the stop member adjacent the opposing sealing surfaces.

In one embodiment according to the present invention, the first jaw member is connected to the bipolar electrosurgical energy source by a pushrod and the second jaw member is connected to the bipolar electrosurgical source by a conductive tube. As can be appreciated, isolating the jaw members in this manner reduces the likelihood of the instrument short circuiting during activation.

In another embodiment, the ratchet is disposed within the fixed handle and at least one complementary interlocking mechanical interface is disposed on the movable handle. Preferably, the ratchet and the complementary interlocking mechanical interface provide at least one interlocking position for maintaining a closure force within the range of about 7 kg/cm² to about 13 kg/cm² between opposing sealing surfaces. Ideally, the closure force is in the range of about 4 kg/cm² to about 6.5 kg/cm². As can be appreciated and as mentioned herein, maintaining the closure force within the above working ranges is a key factor in producing an effective and consistent seal.

The laparoscopic bipolar eleotrosurgical instrument may include a handle having an elongated tube affixed thereto with first and second jaw members attached to a distal end thereof which each include electrically conductive sealing surfaces. The jaw members may be movable from a first position for approximating tissue to at least one subsequent position for grasping tissue therebetween. The handle may have a fixed handle and a handle which is movable relative to the fixed handle to effect movement of the jaw members from the first position to at least one subsequent position for grasping tissue. Advantageously, the sealing surfaces may include a non-stick material for reducing tissue adhesion during the sealing process. The first and second jaw members may be coupled to a source of bipolar electrosurgical energy and a stop may be disposed on at least one of the electrically conductive sealing surfaces to maintain a minimum separation distance between the opposable seal surfaces during sealing.

A ratchet may be disposed on one of the fixed and movable handles and at least one complementary interlocking mechanical interface may be disposed on the other of the fixed and movable handles. Advantageously, the ratchet and the complementary interlocking mechanical interface include at least one interlocking position which maintains a closure force in the range of about 7 kg/cm² to about 13 kg/cm² between opposable seal surfaces.

The non-stick material may be a coating which is deposited on the opposable sealing surfaces. As can be appreciated, this reduces the likelihood of coagulum build-up and sticking. The non-stick coating may be selected from a group of materials consisting of: nitrides and nickel/chrome alloys. Preferably, the non-stick coating may include one of: TiN; ZrN; TiAIN; CrN; nickel/chrome alloys with a Ni/Cr ratio of approximately 5:1; Inconel 600; Ni200; and Ni201.

The opposable sealing surfaces may be manufactured from a non-stick material which is a nickel/chrome alloy. For example, the non-stick material may include nickel/chrome alloys with a Ni/Cr ratio of approximately 5: 1, Inconel 600, Ni200 and Ni201. It is envisioned that these particular materials are advantageous in providing a superior non-stick surface which reduces coagulum build-up and sticking during activation.

Preferably, at least one of the jaw members, handles and elongated tube may include an insulative material disposed thereon which may advantageously be an insulative coating or an insulative sheath.

### BRIEF DESCRIPTION OF THE DRAWINGS

FIG. 1 is a perspective view of a laparoscopic bipolar electrosurgical instrument according to the present invention;
FIG, 2 is a perspective view of the distal end and jaws of the instrument in FIG. 1;
FIG. 3 is an exploded view of the distal end shown in FIG. 2;
FIG. 4 is perspective view of the distal end of the instrument with the jaws removed;
FIG. 5 is another perspective of FIG. 4;
FIG. 6 is a side view of an electrical spring contact; and
FIG. 7 is a front view of the spring contact shown in FIG. 6.

### DETAILED DESCRIPTION OF THE INVENTION

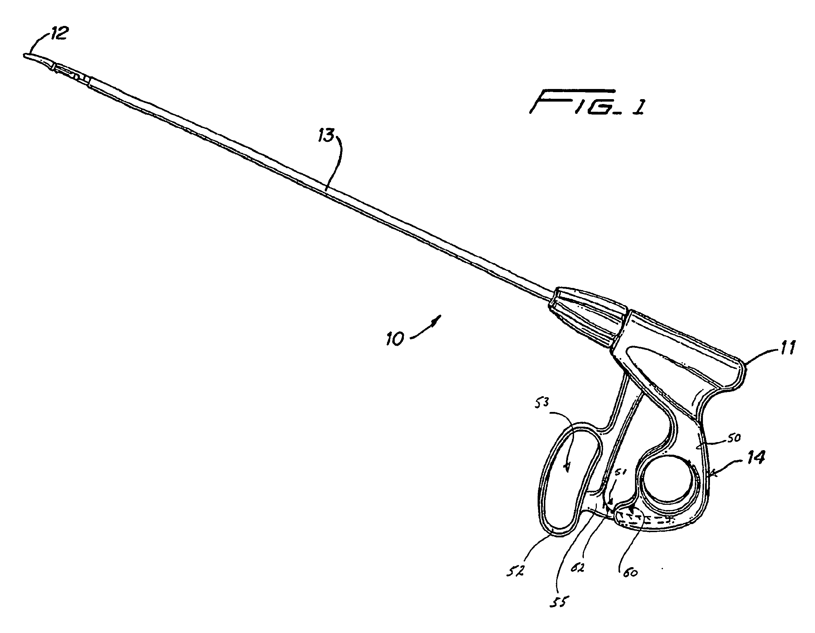

A laparoscopic bipolar electrosurgical instrument 10 is shown in FIG. 1. The instrument 10 has a proximal end 11 with a handle 14 for holding and manipulating the instrument 10. A distal end 12 on the instrument 10 is used for surgical manipulation of tissue. The instrument 10 comprises an elongate tube 13 that is sized to fit through a cannula for laparoscopic operations, and in different embodiments may be sized to fit through a five to ten millimeter cannulas.

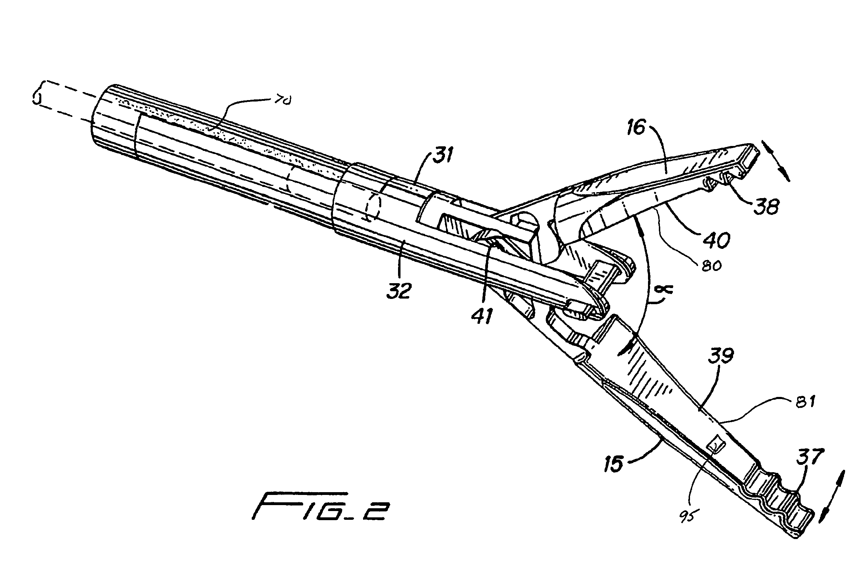

A portion of the distal end 12 of the instrument 10 is shown in FIG. 2. A first jaw 15 and a second jaw 16 are shown in an open position. An angle α is subtended by the jaws 15 and 16. Closing of the jaws 15 and 16 is defined as a reduction of the angle α subtended by the jaws 15 and 16. Similarly, opening of the jaw 15 and 16 is defined as an enlargement of the angle α. The angle α is zero when the jaws 15 and 16 are closed together. The center of rotation for the first jaws 15 is at the first pivot 41, and the center of rotation for the second jaw 16 is at the second pivot 42. The first pivot 41 is located on an outer nose piece 32, and fits in a first pivot hole 43 located on the first flange 18. The second pivot 42 is located on an inner nose piece 31, and fits in a second pivot hole 44 located on the second flange 20.

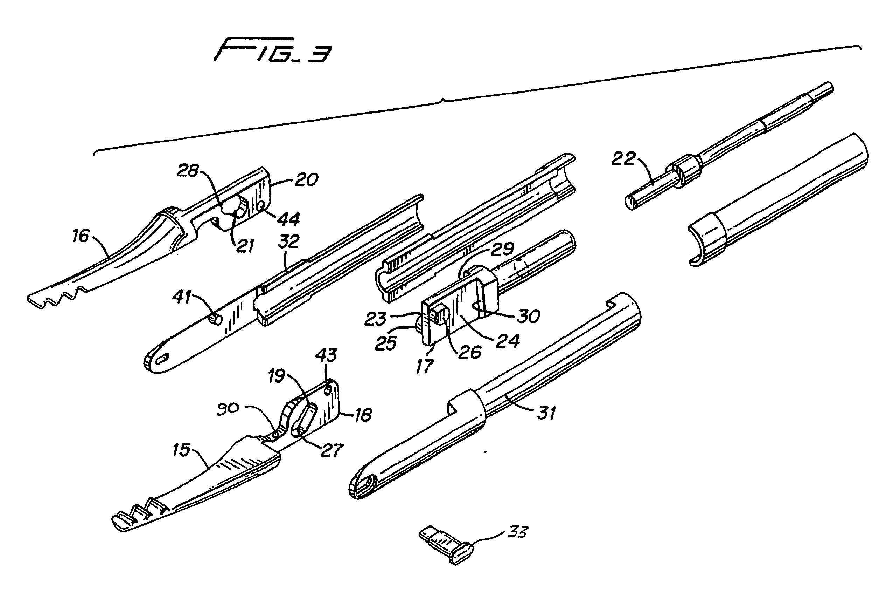

Pieces that comprise the distal end 12 of the instrument 10 are shown in an exploded view in FIG. 3. The first jaw 15 and the second jaw 16 are shown separated from a yoke 17. The first jaw 15 has a first flange 18 and a first slot 19 therewithin. The second jaw 16 has a second flange 20 and a second slot 21 therewithin. Each jaw 15 and 16 is preferably formed from a single piece of stainless steel or other electrically conductive material.

Referring again to FIG. 3, the yoke 17 is attached to a pushrod 22. The yoke 17 is preferably formed from an electrically insulative material such as plastic. A first side 23 of the yoke 17 faces the first flange 18. A second side 24 of the yoke 17 faces the second flange 20. When the yoke 17 is positioned between the flanges 18 and 20. the yoke 17 also acts to electrically insulate the first jaw 15 from the second jaw 16. In this manner, bipolar electrosurgical current can be conducted through tissue grasped by the jaws 15 and 16 without short circuiting between the flanges 18 and 20.

A first pin 25 is located on the first side 23 which movably engages the first slot 19. Similarly, a second pin 26 is located on the second side 24 to movably engage the second slot 21. Each pin and slot combination works as a cam-follower mechanical linkage. Motion of the pushrod 22 moves the yoke 17 causing pins 25 and 26 to slide within their respective slots 19 and 21. The slots 19 and 21 are angled with respect to the distal ends of the jaws 15 and 16 such that the jaws 15 and 16 move in an arcuate fashion toward and away from each other. The pins 25 and 26 are different from the pivots 41 and 42. The pins 25 and 26 provide a force against the walls of the slots 19 and 21, creating a moment about the pivots 41 and 42.

The slots 19 and 21 are arranged such that distal motion of the pushrod 22 causes the jaws 15 and 16 to move together. Distal motion of the pushrod 22 is defined as motion in the direction of the distal end 12 of the instrument 10. Once the jaws 15 and 16 are closed together, the instrument of the present invention holds the jaws 15 and 16 together with a compressive force on the pushrod 22.

One of the advantages of this invention is that shear forces on the pins 25 and 26 can be offloaded to prevent mechanical failure when large forces are being transmitted to the jaws 15 and 16. Each slot 19 and 20 has a cul-de-sac 27 and 28, respectively, as shown in FIG. 3. The first cul-de-sac 27 is an enlargement of the first slot 19 near its distal end. The second cul-de-sac 28 is an enlargement of the second slot 21 near its distal end. The cam-follower motion of the pins 25 and 26 in the slots 19 and 21 will bring the pins 25 and 26 into their respective cul-de-sac 27 and 28. This position of the pins 25 and 26 leaves a very small moment arm between the pins 25 and 26 and the pivots 41 and 42. The yoke 17 has shoulders 29 and 30 that can provide a relatively large moment about the pivots 41 and 42 to effect a high closure force between the jaws 15 and 16 without a high shear force on the pins 25 and 26, as described below.

Once the pins 25 and 26 are in the cul-de-sacs 27 and 28, the force from the yoke is transmitted to the flanges 18 and 20 by a first shoulder 29 and a second shoulder 30. The shoulders 29 and 30 abut the proximal end of the flanges 18 and 20 to cause the jaws 15 and 16 to close together. The pivots 41 and 42 are preferably made of metal and can withstand relatively high shear forces. In contrast, pins 25 and 26 are preferably made of plastic and will break under relatively high shear forces. Thus, the shoulders 29 and 30 provide a moment about the pivots 41 and 42, thereby avoiding the necessity of applying high shear forces to the pins 25 and 26 wherein the moment arm from the pins 25 and 26 would be small. There is an angle α at which the pins 25 and 26 enter their respective cul-de-sacs 27 and 28 and the shoulders 29 and 30 abut the flanges 18 and 20. The angle α at which the forgoing occurs is preferably around three degrees.

The bipolar electrosurgical instrument 10 has first and second poles of alternating potential that are conducted along the instrument 10 and through tissue that is grasped between the jaws 15 and 16. The first pole is conducted from the proximal end 11 toward the distal end 12 along the pushrod 22. The second pole is conducted from the proximal end 11 toward the distal end 12 along the tube 13. The outer surface of the tube 13 is preferably coated with an electrically insulative material. There is also preferably an electrically insulative barrier between the pushrod 22 and the tube 13 to prevent short circuits in the instrument 10.

In the preferred embodiment, the distal end of the instrument 10 comprises an inner nose piece 31 and an outer nose piece 32, as shown in FIG. 2. The inner nose piece 31 is electrically connected with the pushrod 22, while the outer nose piece is electrically connected with the tube 13. The inner nose piece 31 and the outer nose piece 32 capture the yoke 17, along with the first and second flanges 18 and 20, as shown in FIG. 2. The yoke 17 moves axially, along an axis defined by the tube 13, in a space between the inner and outer nose pieces 31 and 32. A spacer stake 33 maintains the separation of the nose pieces 31 and 32 at their distal ends. The nose pieces 31 and 32 provide lateral support for the flanges 18 and 20 to help ensure that the pins 25 and 26 remain within the slots 19 and 21, respectively.

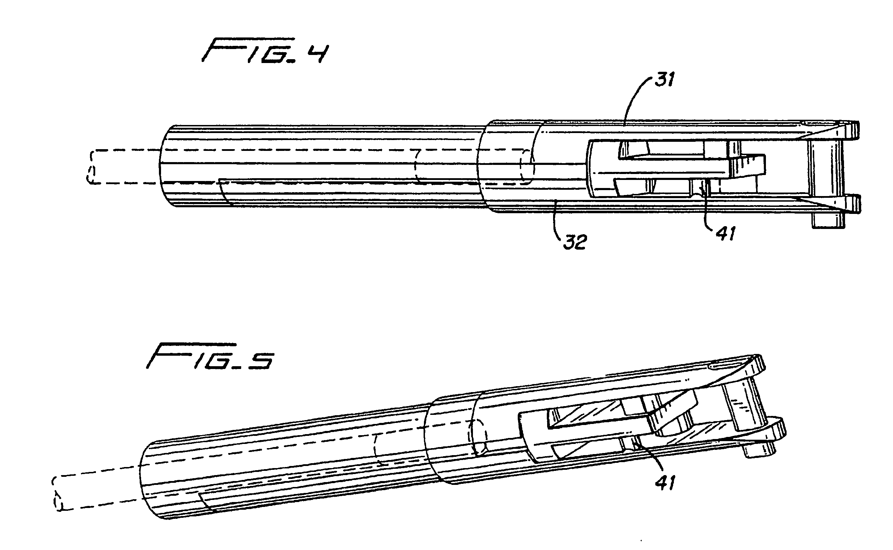

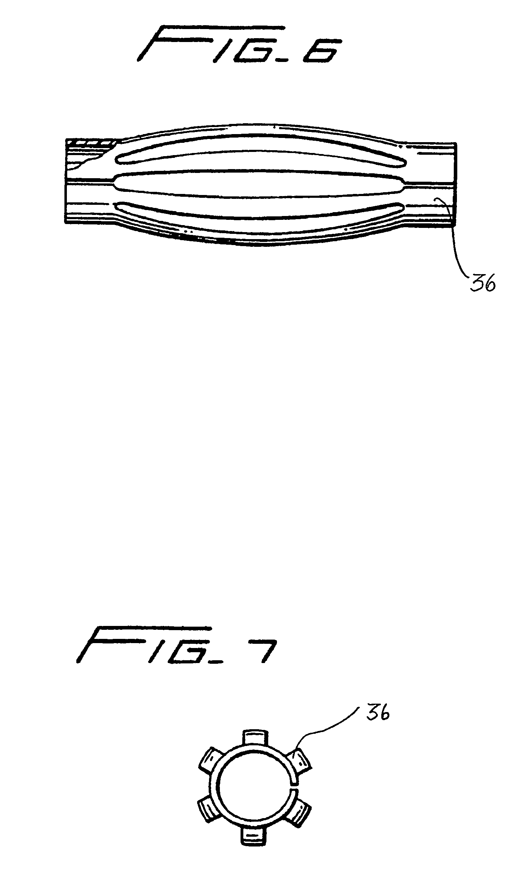

The preferred embodiment also comprises an inner insulator 34 and an outer insulator 35 for maintaining electrical insulation between the poles. The outer insulator 35 is seated between the tube 13 and the inner nose 31, as shown in FIGS. 2 and 4. The inner insulator 34 is seated between the tube 13 and the pushrod 22. In this manner, the outer nose piece 32 can provide electrical continuity between the tube 13 and the second jaw 16, while the inner nose piece 34 can provide electrical continuity between the pushrod 22 and the first jaw 15. Since the pushrod 22 is slidably mounted within the tube 13, the preferred embodiment has a spring contact 36, as shown in FIGS. 6 and 7, which is mounted on the pushrod 22 to maintain an electrical connection with the inner nose piece 34 during axial motion.

The first and second jaws 15 and 16 each have ridges 37 and 38 at their distal ends that preferably nest together. The jaws 15 and 16 also have seal surfaces 39 and 40, as shown in FIG. 2. The width of the seal surfaces 39 and 40 is a parameter that affects the quality of the surgical outcome. The closure force between the jaws 15 and 16 varies along the length of the seal surfaces 39 and 40, with the largest force at the distal tip and the smallest force at the proximal end of the seal surfaces 39 and 40. It is known that the amount of pressure exerted on the tissue depends on the surface area of the tissue that is in contact with the seal surfaces. In the one embodiment, the width of each seal surface, e.g., 39, is in the range of about 2 to about 5 millimeters, and preferably 4 millimeters width, while the length of each seal surface 39 and 40 is preferably in the range of about 10 to 30 millimeters.

It has been found through experimentation that good vessel sealing results are obtained when the closure force in grams divided by the width in millimeters is in the range of about 400 to 650 grams per millimeter of seal surface width. Since the closure force varies with the length of the seal surfaces 39 and 40, it has been found to be advantageous to taper the width of the seal surfaces 39 and 40 along their length, with the widest width at the proximal end and the narrowest width at the distal end. For example, if the width of the seal surface 39, 40 is 4 millimeters, the closure force is preferably in the range of about 1600 grams to about 2600 grams This design allows the jaws 15 and 16 to apply a relatively constant closure force per unit width, preferably 525 grams per millimeter width which yields a closure force of 2100 grams for a 4 millimeter width seal surface 39, 40.

In one embodiment, the handle 14 includes a fixed handle 50 having a channel 51 defined therein which slidingly receives a movable handle 52. Movable handle 52 includes a handgrip 53 defined therein which allows a user to move handle 52 relative to fixed handle 50. Movable handle 52 also includes a flange 55 having a series of grooves 62 defined therein which mechanically inter-engage a corresponding ratchet 60 disposed within channel 51. Preferably, the ratchet 60 and groove 62 are dimensioned such that successive ratchet positions will yield pressures within a predetermined working range of about 7 kg/cm² to about 13 kg/cm². In one embodiment, the successive ratchet positions are two millimeters apart.

Experimental results in tissue studies suggest that the magnitude of pressure exerted on the tissue by the seal surfaces 39 and 40 is important in assuring a proper surgical outcome. Tissue pressures within a working range of about 3 kg/cm² to about 16 kg/cm² and, preferably, within a working range of 7 kg/cm² to 13 kg/cm² have been shown to be effective for sealing arteries and vascular bundles. Tissue pressures within the range of about 4 kg/cm² to about 6.5 kg/cm² have proven to be particularly effective in sealing arteries and tissue bundles.

A method of making a laparoscopic bipolar electrosurgical instrument 10 is also herein described. The method comprises the step of forming a first jaw 15 having a first flange 18 with a first slot 19, and a second jaw 16 having a second flange 20 with a second slot 21. The jaws 15 and 16 are preferably formed in a casting process, although it is also possible to machine the jaws 15 and 16 from stock. The casting process may include injecting powdered metal under pressure into a mold, and then applying heat.

Other steps in the method include attaching a yoke 17 to a pushrod 22, and electrically insulating the first flange 18 from the second flange 20 with the yoke 17. The yoke 17 is preferably an injection molded plastic part with features including a first shoulder 29 and a second shoulder 30.

During assembly of the distal portion of the instrument 10, steps in the method include engaging a first pin 25 with the first slot 19, and engaging a second pin 26 with the second slot 21. The slots 19 and 21 are shaped such that a subtended angle α between the first and second jaws 15 and 16 decreases with distal motion of the pushrod 17. The slots 19 and 20 are formed with cul-de-sacs 27 and 28 positioned to relieve shear stresses on the first and second pins 25 and 26 at the subtended angle α approximately where the first and second shoulders 29 and 30 engage the first and second flanges 18 and 20.

Further steps in the method comprise: surrounding at least a portion of the pushrod 22 with an electrically conductive tube 13; electrically insulating the tube 13 from the pushrod 22; electrically connecting an inner nose piece 31 to the pushrod 22, and electrically connecting an outer nose piece 32 to the tube 13, wherein the inner nose piece 31 and the outer nose piece 32 capture the yoke 17 along with the first and second flanges 18 and 20 to conduct bipolar electrosurgical current to the first and second jaws 15 and 16. In the preferred embodiment, there is a step of electrically connecting the pushrod 22 and the inner nose piece 31 with a spring contact 36.

The method of making the instrument 10, in some embodiments, includes the steps of tapering the width of the seal surfaces 39 and 40 along the length of each of the first and second jaws 15 and 16.

An electrically insulative coating 70 may be included to substantially cover the elongated tube 13 to protect the surgeon against electrical arcs. Other parts of the instrument may also be protected by the insulative coating 70. An insulative sheath may also be used to cover tube 13 or other components of the instrument 10, e.g., the proximal end 11, handles 50, 52 and the outer surfaces (non-opposing surfaces) of the jaw members 15, 16.

In other aspects, which do not form part of the present invention, the outer surface of the jaw members 15 and 16 may include a nickel-based material, coating, stamping, metal injection molding which is designed to reduce adhesion between the jaw members (or components thereof) with the surrounding tissue during activation and sealing. Moreover, other components such as the tube 13 and handles 50, 52 may also be coated with the same or a different "non-stick" material. Preferably, the non-stick materials are of a class of materials that provide a smooth surface to prevent mechanical tooth adhesions.

The tissue sealing surfaces 39 and 40 of the jaw members 15 and 16, respectively, may be manufactured from one (or a combination of one or more) of the following "non-stick" materials: nickel-chrome, chromium nitride, MedCoat 2000 manufactured by The Electrolizing Corporation of OHIO, Inconel 600 and tin-nickel. For example, high nickel chrome alloys and Ni200, Ni201 (~100% Ni) may be made into electrodes or sealing surfaces by metal injection molding, stamping, machining or any like process.

In addition, these materials preferably may include an optimal surface energy for eliminating sticking due in part to surface texture and susceptibility to surface breakdown due electrical effects and corrosion in the presence of biologic tissues. These materials may exhibit superior non-stick qualities over stainless steel and should be utilized on the instrument in areas where the exposure to pressure and RF energy can create localized "hot spots" more susceptible to tissue adhesion. As can be appreciated, reducing the amount that the tissue "sticks" during sealing improves the overall efficacy of the instrument.

The tissue sealing surfaces 39 and 40 may also be "coated" with one or more of the above materials to achieve the same result, i.e., a "non-stick surface". For example, Nitride coatings (or one or more of the other above-identified materials) may be deposited as a coating on another base material (metal or nonmetal) using a vapor deposition manufacturing technique.

One particular class of materials disclosed herein has demonstrated superior non-stick properties and, in some instances, superior seal quality. For example, nitride coatings which include, but not are not limited to: TiN, ZrN, TiAIN, and CrN are preferred materials used for non-stick purposes. CrN has been found to be particularly useful for non-stick purposes due to its overall surface properties and performance. Other classes of materials have also been found to reducing overall sticking. For example, high nickel/chrome alloys with a Ni/Cr ratio of approximately 5:1 have been found to significantly reduce sticking in bipolar instrumentation. One particularly useful non-stick material in this class is Inconel 600. Bipolar instrumentation having electrodes made from or coated with Ni200, Ni201 (~100% Ni) also showed improved non-stick performance over typical bipolar stainless steel electrodes.

It has been found experimentally that local current concentrations can result in an uneven tissue effect, and to reduce the possibility of this outcome, each seal surface 39 and 40 may include a radiused edge 80, 81. As mentioned above, a tapered seal surface 39 and 40 has been shown to be advantageous in certain embodiments because the taper allows for a relatively constant pressure on the tissue along the length of the seal surfaces 39 and 40. The width of the seal surfaces 39 and 40 may be adjusted to assure that the closure force divided by the width is approximately constant along the length.

In one embodiment, a stop 90, made from insulative material, is located in the instrument to maintain a minimum separation of at least about 0.03 millimeters between the seal surfaces 39 and 40, as shown in FIG. 3. Preferably, the stop maintains a minimum separation distance in the range of about 0.03 millimeters to about 0.16 millimeters. The stop 90 reduces the possibility of short circuits between the seal surfaces 39 and 40. It is envisioned that stop 90 may be positioned proximate the pivots 41 and 42, proximate the stake 33 or adjacent the opposable seal surfaces 39 and 40.

In another embodiment, the instrument 10 includes a second or alternative stop 95 which is designed to maintain a minimum separation of at least 0.03 millimeters between the seal surfaces 39 and 40, as shown in FIG. 2. Preferably, the stop 90 and/or the stop 95 maintains a separation distance within the range of about 0.03 millimeters to about 0.16 millimeters. A plurality of stops 90 and/or 95 (or various patterns of stops 90, 95) may also be utilized to accomplish this purpose.

## Claims

1. A laparoscopic bipolar electrosurgical instrument (10) for sealing tissue, comprising:
a handle (14) having an elongated tube (13) affixed thereto, the tube including first and second jaw members (15, 16) attached to a distal end thereof, the first and second jaw members being movable from a first position in which the first and second jaw members are disposed in spaced relation relative to one another to at least one subsequent position for grasping and sealing tissue held therebetween, each of the first and second jaw members including a flange (18, 20) and an electrically conductive sealing surface (39, 40) with a closure force in the range of about 3 kg/cm² to about 16 kg/cm² maintained between the opposed sealing surfaces of the first and second jaw members, the handle including a fixed handle (50) and a movable handle (52), the movable handle being movable relative to the fixed handle to effect movement of the first and second jaw members from the first position to the at least one subsequent position for grasping and sealing tissue held therebetween;
wherein the first and second jaw members are adapted to connect to a source of electrosurgical energy such that the opposable seal surfaces are capable of conducting electrosurgical energy through tissue held therebetween;
a stop (90, 95) adapted to maintain a minimum separation distance of at least 0.03 millimeters between the opposed sealing surfaces; and
a yoke (17) having pins (25, 26) for approximating the jaws and shoulders (29, 30), wherein the shoulders can press on the respective jaw flanges to effect the respective closure force between the sealing surfaces without imposing high shear forces on the pins.

2. A laparoscopic bipolar electrosurgical instrument according to claim 1, further comprising
a pushrod (22) for connecting the first jaw member to a source of electrosurgical energy; and
a conductive tube for connecting the second jaw member to the source of electrosurgical energy.

3. A laparoscopic bipolar electrosurgical instrument according to any preceding claim, wherein the closure force is maintained by means of a ratchet (60) disposed within the fixed handle and at least one complementary interlocking mechanical interface (62) disposed on the movable handle, the ratchet and the complementary interlocking mechanical interface providing at least one interlocking position for maintaining the closure force.

4. A laparoscopic bipolar electrosurgical instrument according to any preceding claim, wherein the closure force is in the range of about 7 kg/cm² to about 13 kg/cm².

5. A laparoscopic bipolar electrosurgical instrument according to any preceding claim, wherein the closure force is in the range of about 4 kg/cm² to about 6.5 kg/cm².

6. A laparoscopic bipolar electrosurgical instrument according to any preceding claim, wherein the stop is disposed on at least one of the sealing surfaces.

7. A laparoscopic bipolar electrosurgical instrument according to any preceding claim, wherein the stop is disposed adjacent to at least one of the sealing surface.

8. A laparoscopic bipolar electrosurgical instrument according to any preceding claim, wherein the stop maintains a minimum separation distance between sealing surface in the range of about 0.03 millimeters to about 0.16 millimeters.

9. A laparoscopic bipolar electrosurgical instrument according to any one of the preceding claims, wherein the yoke is made from an electrically insulative material, and is positioned inbetween the first and second jaw members.

## Patentansprüche

1. Laparoskopisches bipolares elektrochirurgisches Instrument (10) zum Verschließen von Gewebe, mit:
einem Griff (14) mit einem länglichen daran befestigten Rohr (13), wobei das Rohr erste und zweite Backenelemente (15, 16) umfasst, die an einem distalen Ende davon angebracht sind, wobei die ersten und zweiten Backenelemente von einer ersten Position, in der die ersten und zweiten Backenelemente relativ zueinander beabstandet angeordnet sind, in zumindest eine nachfolgende Position, zum Ergreifen und Verschließen von dazwischen gehaltenem Gewebe, bewegbar sind, wobei jedes der ersten und zweiten Backenelemente einen Flansch (18, 20) und eine elektrisch leitfähige Verschlussoberfläche (39, 40) umfasst, mit einer Schließkraft, in dem Bereich von ungefähr 3 kg/cm² bis ungefähr 16 kg/cm², die zwischen den gegenüberliegenden Verschlussoberflächen der ersten und zweiten Backenelemente beibehalten wird, wobei der Griff einen unbeweglichen Griff (50) und einen bewegbaren Griff (52) umfasst, der bewegbare Griff relativ zu dem unbeweglichen Griff bewegbar ist, um eine Bewegung der ersten und zweiten Backenelemente von der ersten Position in die zumindest eine nachfolgende Position, zum Ergreifen und Verschließen von dazwischen gehaltenem Gewebe, zu bewirken;
wobei die ersten und zweiten Backenelemente geeignet sind mit einer Quelle von elektrochirurgischer Energie verbunden zu werden, derart, dass die gegenüberlegbaren Verschlussoberflächen elektrochirurgische Energie durch das dazwischen gehaltene Gewebe leiten können;
einem Anschlag (90, 95), der geeignet ist einen minimalen Trennungsabstand von zumindest 0,03 Millimeter zwischen den gegenüberliegenden Verschlussoberflächen beizubehalten; und
einem Joch (17) mit Stiften (25, 26) zum Annähern der Backen und Schultern (29, 30), wobei die Schultern auf die jeweiligen Backenflansche drücken können, um die jeweilige Schließkraft zwischen den Verschlussoberflächen zu bewirken, ohne den Stiften große Scherkräfte aufzuerlegen.

2. Laparoskopisches bipolares elektrochirurgisches Instrument nach Anspruch 1, ferner mit:
einem Schubstab (22) zum Verbinden des ersten Backenelements mit einer Quelle von elektrochirurgischer Energie; und
einem leitfähigen Rohr zum Verbinden des zweiten Backenelements mit der Quelle von elektrochirurgischer Energie.

3. Laparoskopisches bipolares elektrochirurgisches Instrument nach einem der vorhergehenden Ansprüche, bei dem die Schließkraft mittels einer Ratsche (60), die innerhalb des unbeweglichen Griffs angeordnet ist, und zumindest einer komplementären mechanischen Verriegelungsschnittstelle (62) beibehalten wird, die an dem bewegbaren Griff angeordnet ist, wobei die Ratsche und die komplementäre mechanische Verriegelungsschnittstelle zumindest eine Verriegelungsposition zur Beibehaltung der Schließkraft vorsehen.

4. Laparoskopisches bipolares elektrochirurgisches Instrument nach einem der vorhergehenden Ansprüche, bei dem die Schließkraft in dem Bereich von ungefähr 7 kg/cm² bis ungefähr 13 kg/cm² liegt.

5. Laparoskopisches bipolares elektrochirurgisches Instrument nach einem der vorhergehenden Ansprüche, bei dem die Schließkraft in dem Bereich von ungefähr 4 kg/cm² bis ungefähr 6,5 kg/cm² liegt.

6. Laparoskopisches bipolares elektrochirurgisches Instrument nach einem der vorhergehenden Ansprüche, bei dem der Anschlag an zumindest einer der Verschlussoberflächen angeordnet ist.

7. Laparoskopisches bipolares elektrochirurgisches Instrument nach einem der vorhergehenden Ansprüche, bei dem der Anschlag an zumindest eine der Verschlussoberflächen angrenzend angeordnet ist.

8. Laparoskopisches bipolares elektrochirurgisches Instrument nach einem der vorhergehenden Ansprüche, bei dem der Anschlag einen minimalen Trennungsabstand zwischen den Verschlussoberflächen in dem Bereich von ungefähr 0,03 Millimeter bis ungefähr 0,16 Millimeter beibehält.

9. Laparoskopisches bipolares elektrochirurgisches Instrument nach einem der vorhergehenden Ansprüche, bei dem das Joch aus einem elektrisch isolierenden Material hergestellt ist, und zwischen den ersten und zweiten Backenelementen positioniert ist.

## Revendications

1. Instrument électrochirurgical bipolaire laparoscopique (10) pour le scellement de tissu, comprenant:
une poignée (14) présentant un tube oblong (13) fixé à celle-ci, le tube ayant des premier et second éléments de mâchoire (15, 16)fixés à son extrémité distale, les premier et second éléments de mâchoire étant déplaçables d'une première position dans laquelle les premier et second éléments de mâchoire sont disposés selon une relation espacée l'un par rapport à l'autre à au moins une position suivante pour saisir et sceller le tissu tenu entre eux, chacun des premier et second éléments de mâchoire incluant une bride (18, 20) et une surface de scellement électriquement conductrice (39, 40) avec une force de fermeture dans la plage d'environ 3 kg/cm² à environ 16 kg/cm² maintenue entre les surfaces de scellement opposées des premier et second éléments de mâchoire, la poignée comprenant une poignée fixe (50) et une poignée mobile (52), la poignée mobile étant déplaçable relativement à la poignée fixe pour entraîner un mouvement des premier et second éléments de mâchoire de la première position au moins à la position suivante pour saisir et sceller le tissu tenu entre eux;
où les premier et second éléments de mâchoire sont aptes à être reliés à une source d'énergie électrochirurgicale de façon que les surfaces de scellement opposables soient aptes à conduire l'énergie électrochirurgicale à travers le tissu tenu entre elles;
une butée (90, 95) apte à maintenir une distance de séparation minimale d'au moins 0,03 millimètres entre les surfaces de scellement opposées; et
une culasse (17) ayant des axes (25, 26) pour rapprocher les mâchoires et des épaulements (29, 30), où les épaulements peuvent exercer une pression sur les brides de mâchoire respectives pour produire la force de fermeture respective entre les surfaces de scellement sans imposer des forces de cisaillement élevées aux axes.

2. Instrument électrochirurgical bipolaire laparoscopique selon la revendication 1, comprenant en outre:
une tige de poussée (22) pour relier le premier élément de mâchoire à une source d'énergie électrochirurgicale; et
un tube conducteur pour relier le second élément de mâchoire à la source d'énergie électrochirurgicale.

3. Instrument électrochirurgical bipolaire laparoscopique selon l'une des revendications précédentes, où la force de fermeture est maintenue au moyen d'un cliquet (60) disposé dans la poignée fixe et au moins une interface mécanique d'interverrouillage complémentaire (62) disposée sur la poignée mobile, le cliquet et l'interface mécanique d'interverrouillage complémentaire réalisant au moins une position d'interverrouillage pour maintenir la force de fermeture.

4. Instrument électrochirurgical bipolaire laparoscopique selon l'une des revendications précédentes, où la force de fermeture se situe dans la plage d'environ 7 kg/cm² à environ 13 kg/cm².

5. Instrument électrochirurgical bipolaire laparoscopique selon l'une des revendications précédentes, où la force de fermeture se situe dans la plage d'environ 4 kg/cm² à environ 6,5 kg/cm².

6. Instrument électrochirurgical bipolaire laparoscopique selon l'une des revendications précédentes, où la butée est disposée sur au moins l'une des surfaces de scellement.

7. Instrument électrochirurgical bipolaire laparoscopique selon l'une des revendications précédentes, où la butée est disposée pour qu'elle soit adjacente à au moins l'une des surfaces de scellement.

8. Instrument électrochirurgical bipolaire laparoscopique selon l'une des revendications précédentes, où la butée maintient une distance de séparation minimale entre les surfaces de scellement dans la plage d'environ 0,03 millimètre à environ 0,16 millimètre.

9. Instrument électrochirurgical bipolaire laparoscopique selon l'une des revendications précédentes, où la culasse est réalisée à partir d'un matériau électriquement isolant et est positionnée entre les premier et second éléments de mâchoire.
